# EUROPEAN PATENT APPLICATION

(11) **EP 4 089 160 A1**
(43) Date of publication of application: **16.11.2022**
(21) Application number: 20912613.5
(22) Date of filing: 22.09.2020
(51) Int. Cl.: C12M 1/33, C12M 1/34, G01N 1/28

(54) **CELL EXTRUDER AND CELL EXTRUSION METHOD**

(30) Priority: 09.01.2020 KR 20200003134
(71) Applicant: MDimune Inc., Seoul 04790 (KR)
(72) Inventor: KIM, Se Hee, Seoul 04728 (KR); LEE, Ji Eun, Seoul 01784 (KR); BAE, Shin Gyu, Seongnam-si Gyeonggi-do 13547 (KR); LEE, Ji Hye, Incheon 22698 (KR); PARK, Jin Hee, Paju-si Gyeonggi-do 10872 (KR)
(74) Representative: Boult Wade Tennant LLP
(86) International application number: PCT/KR2020/012742
(87) International publication number: WO 2021/141207

(57) **Abstract**

A cell extruder of the present invention comprises: a pressure vessel in which a sample is dispensed; a regulator for adjusting a set pressure of nitrogen gas injected into the pressure vessel and then maintaining a constant pressure; an input valve for opening and closing the injection of nitrogen gas into the pressure vessel; an exhaust valve for removing the internal pressure of the pressure vessel; a filter holder which is provided with a membrane filter soaked with a reagent, so that the sample in the pressure vessel is fed through by the nitrogen gas and crushed into extracellular vesicles; and a collection container in which the extruded sample is stored. According to the present invention, pressure can be freely adjusted by the regulator. That is, the flow rate of the membrane according to pressure can be controlled, thus allowing the validation of a biopharmaceutical production method using an extracellular vesicle extruder. In addition, during cell extrusion, a sintered disc can be removed and cells can be extruded by using only a membrane. Vesicles can be produced within a fine pressure adjustment range (at a fine low pressure at which the membrane is not torn) in which no sintered disc is required.

## Description

### [Technical Field]

The present invention relates to a cell extruder and a cell extrusion method, and more particularly, to a cell extruder and a cell extrusion method capable of extruding extracellular vesicles from cells.

### [Background Art]

An extracellular vehicle (EV) refers to a substance of a double lipid membrane structure that is generally secreted from cells. In general, there are largely three types of extracellular vesicles (EVs), which consist of microvesicles, exosomes, and apoptic bodies, and are divided according to their origins and biogenesis, efficacy, and roles.

These extracellular vesicles have been actively developed in the field of diagnosis and treatment in recent years.

However, the amount of extracellular vesicles secreted from the cells is quite small, so that there are many limitations in development, commercialization, and industrialization for clinical use. Recently, research for mimicking these extracellular vesicles is actively being conducted.

The applicant of the present invention holds a technology for artificially manufacturing extracellular vesicles from cells (Patent Registration No. 1314868, "Microvesicles derived from mammalian nucleated cells and use thereof'), and has developed therapeutic agents using artificial exosomes derived from stem cells and immune cells.

However, there is no cell-specific extruder in the related art, and a conventional liposome extruder has the following problems.

First, there is a problem in that it is not easy to control a micropressure.

That is, if the micropressure is not controlled, when the extracellular vesicles are manufactured, the vesicles are not well formed and the topology of vesicles derived from cells is not maintained, so that it is difficult to expect a therapeutic effect exhibited from extracellular vesicles.

Second, the cell capacity of the filter size of the extruded part is unstable.

That is, when the extracellular vesicles are manufactured, vesicles and cell debris are mixed, and thus a membrane receiving area is determined according to the number of cells. However, in a conventional extruder, since the membrane receiving area is not suitable for vesicle production, the membrane is clogged during vesicle production, so that the manufacturing process is not easy.

Third, it is not easy to clean the extruder, which causes contamination.

That is, the conventional extruder is manufactured as a screw type and does not meet a GMP compliance standard.

Fourth, in a process of crushing cells, an extrusion path was clogged due to structural problems of a sintered disc and an extruder.

That is, in the case of a sintered disc supporting the membrane, when the cells are extruded, the cell receiving area is affected and clogged, so that there is a disadvantage that it is not easy when manufacturing the vesicles.

Fifth, according to the notification of the Ministry of Food and Drug Safety, the use of cells needs to be handled inside (zone 100) of a biological safety cabinet (BSC), but inconvenience in use was caused due to the size of the extruder of the related art.

Accordingly, the development of improved cell extruder and cell extrusion method has been required.

### [Disclosure]

### [Technical Problem]

The present invention has been devised to solve the above-mentioned problems in the prior art, and an object of the present invention is to provide a cell extruder and a cell extrusion method capable of freely controlling a pressure.

Yet another object of the present invention is to provide a cell extruder and a cell extrusion method capable of stabilizing a cell capacity.

Still another object of the present invention is to provide a cell extruder and a cell extrusion method capable of being easily cleaned.

Still another object of the present invention is to provide a cell extruder and a cell extrusion method capable of preventing the clogging of an extrusion path.

Still another object of the present invention is to provide a cell extruder and a cell extrusion method capable of being easily handled in a biological safety cabinet.

### [Technical Solution]

One aspect of the present invention provides a cell extruder including: a pressure vessel in which a sample is dispensed; a regulator for adjusting a set pressure of gas injected into the pressure vessel and then maintaining a constant pressure; an input valve for opening and closing the injection of gas into the pressure vessel; an exhaust valve for removing the internal pressure of the pressure vessel; a filter holder which is provided with a membrane filter, so that a sample in the pressure vessel is fed through by the gas and crushed into small pieces; and a collection container in which the extruded sample is stored.

The cell extruder may further include a pressure gauge for measuring the pressure of the pressure vessel.

The cell extruder may further include a diaphragm valve that opens the flow path to the filter holder only when the gas pressure acts on a diaphragm to reach the set pressure.

In the cell extruder, the pressure vessel, the regulator, the input valve, the exhaust valve, the filter holder, the collection container, the pressure gauge, and the diaphragm valve may be disassembled and assembled from and with adjacent components by clamps.

In the cell extruder, the membrane filters may be provided in the order of decreasing the pore size.

A plurality of membrane filters may be provided in the cell extruder in the order of decreasing the pore size at the same time or sequentially replaced in the order of decreasing the size.

The pressure vessel and the filter holder may be connected to each other by a pipe provided with a nozzle, and a diameter ratio of the nozzle and the filter holder may be important, and preferably, may be 1 : 3 to 1 : 30.

The gas is not limited to the type thereof, but may be at least one selected from the group consisting of nitrogen gas, compressed air, carbon dioxide, and inert gas.

In addition, the cell extruder may be applied to a sample containing cells, and may also be used to extrude cell-derived extracellular vesicles into smaller sizes.

Another aspect of the present invention provides a manufacturing method of cell-derived extracellular vesicles including applying the cell extruder to cells.

The method may include 1) supplying a sample containing cells to the cell extruder; 2) extruding the sample by driving the cell extruder of step 1); and 3) obtaining the sample extruded in step 2).

Step 2) may include i) dispensing the sample to a pressure vessel; ii) supplying gas to the pressure vessel by opening the input valve; and iii) passing the sample through a membrane filter in a filter holder from the pressure vessel through a nozzle in a pipe.

The gas may be supplied at a pressure of 20 to 100 psi.

Step iii) may include passing the sample through the membrane filters sequentially in the order of decreasing the pore size, in which the membrane filters may be provided in the filter holder at the same time or replaced sequentially.

Yet another aspect of the present invention provides cell-derived extracellular vesicles manufactured by the method. The cell-derived extracellular vesicles may be used in pharmaceuticals, food compositions, and the like, and may also be used in a drug delivery system (DDS).

### [Advantageous Effects]

According to the cell extruder of the present invention, it is possible to freely control a pressure by a regulator. That is, the flow rate of the membrane according to a pressure can be controlled, thus allowing the validation of a biopharmaceutical production method using an extracellular vesicle extruder.

In addition, there is an advantage in that the vesicle production is increased and the topology of the vesicle is maintained by stabilizing the cell capacity during cell extrusion to facilitate the formation of the vesicle.

In addition, the extruder can be separated through clamps provided between the components of the present invention, so that cleaning is easy.

In addition, unlike the related art, after removing the sintered disc, the number of cells, a micropressure, and a membrane capacity range are established to prevent the clogging of the extrusion path.

That is, during cell extrusion, a sintered disc can be removed, cells can be extruded by using only a membrane, and vesicles can be produced within a micropressure adjustment range (at a fine low pressure at which the membrane is not torn) in which no sintered disc is required.

In addition, the cell extruder of the present invention is implemented in a compact size, so that it is easy to be handled in a limited space, a biological safety cabinet.

### [Description of Drawings]

FIG. 1 is an exploded view of components of a cell extruder according to the present invention.
FIG. 2 is a schematic diagram illustrating an assembling state of FIG. 1.

### [Best Mode of the Invention]

Hereinafter, a preferred exemplary embodiment of the present invention will be described in detail with reference to the accompanying drawings.

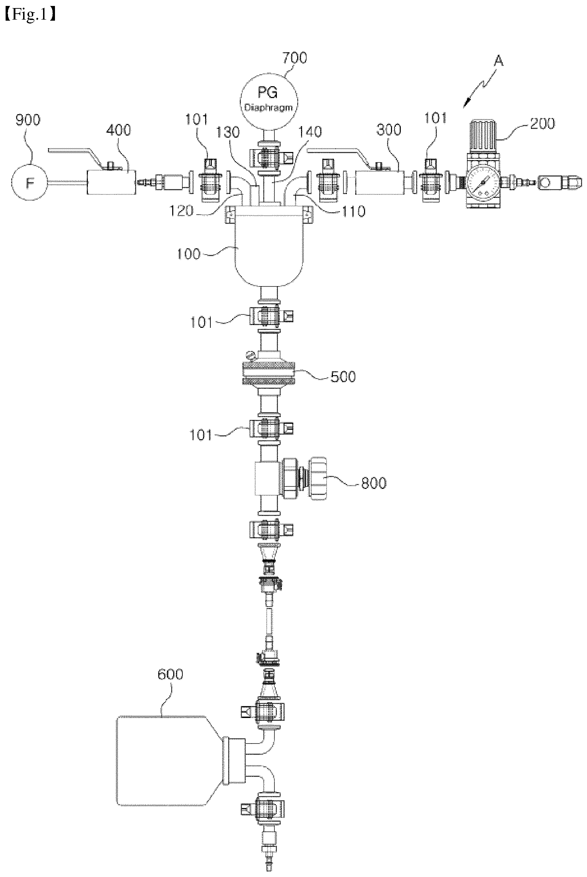

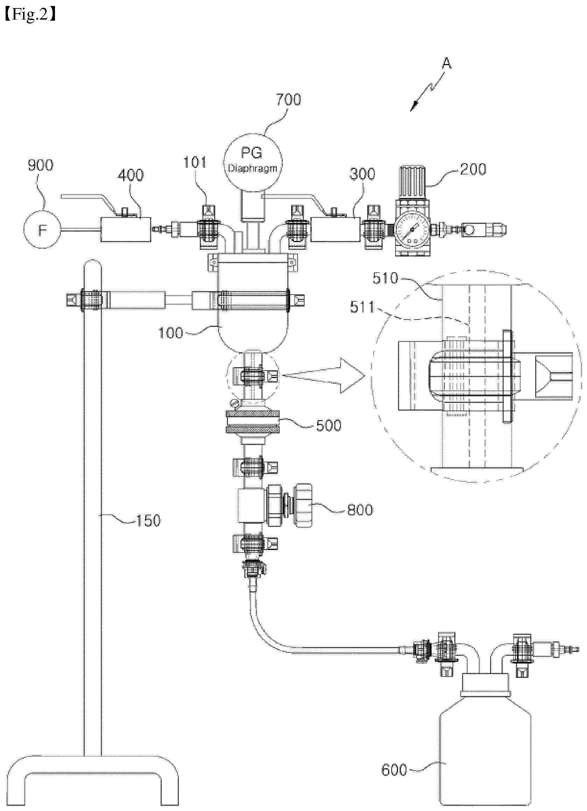

FIG. 1 is an exploded view of components of a cell extruder according to the present invention and FIG. 2 is a schematic diagram illustrating an assembling state of FIG. 1.

As illustrated in FIGS. 1 and 2, a cell extruder A according to the present invention is configured by a pressure vessel 100 in which a sample is dispensed; a regulator 200 for adjusting a set pressure of gas injected into the pressure vessel 100 and then maintaining a constant pressure; an input valve 300 for opening and closing the injection of gas into the pressure vessel 100; an exhaust valve 400 for removing the internal pressure of the pressure vessel; a filter holder 500 which is provided with a membrane filter soaked with a reagent, so that a sample in the pressure vessel 100 is fed through by the gas and crushed into extracellular vesicles; and a collection container 600 in which the extruded sample is stored.

The pressure vessel 100 is clamped to a stand 150 and then maintained in an upright form, and includes an input line 110 to which gas is inputted, an exhaust line 120 for discharging the internal pressure of the pressure vessel 100, an injection line 130 for injecting the sample into the pressure vessel 100, and a pressure measuring line 140 for measuring the internal pressure of the pressure vessel 100.

The regulator 200 is to maintain the constant pressure after adjusting the input pressure to the set pressure. Specifically, the regulator 200 is configured to obtain an extrudate of uniform quality by injecting the sample into the pressure vessel 100 while maintaining the constant pressure after adjusting the gas of a set pressure or higher to the set pressure and applying uniform pressure.

The input valve 300 is connected to the input line 110, and the exhaust valve 300 is a well-known opening/closing valve connected to the exhaust line 120 to open and close the flow path. The detailed description of the structure thereof will be omitted and the operation thereof will be described in an operation description to be described below.

The filter holder 500 has an open/close structure and is formed to accommodate a membrane filter therein. At this time, the membrane filter crushes the sample fed through the gas from the pressure vessel 100 into the extracellular vesicles.

The gas is supplied to the filter holder 500 through a pipe 510 provided with a nozzle 511, and a diameter ratio between the nozzle 511 and the filter holder 500 is 1 : 3 to 1 : 30.

The membrane filters may be provided in the order of decreasing the pore size, and may be simultaneously provided in the cell extruder or sequentially replaced.

The collection container 600 has a container shape with a part opened, and stores the extruded sample.

In addition, a pressure gauge 700 for measuring the pressure of the pressure vessel 100 is further provided to visually display whether the internal pressure of the pressure vessel maintains the set pressure. In this case, the pressure gauge 700 may be added with a function of generating an alarm when the pressure is greater than or less than the set pressure.

In addition, a diaphragm valve 800 that opens the flow path to the filter holder 500 only when the gas pressure acts on a diaphragm to reach the set pressure is further provided. That is, when the pressure of the gas feeding the sample does not reach the set pressure, the sample is restricted from moving to the filter holder 500.

The gas may be at least one selected from the group consisting of nitrogen gas, compressed air, carbon dioxide, and inert gas.

In addition, an exhaust filter 900 is provided at a discharge end of the exhaust valve 400.

Meanwhile, the pressure vessel 100, the regulator 200, the input valve 300, the exhaust valve 400, the filter holder 500, the collection container 600, the pressure gauge 700, the diaphragm valve 800, and the exhaust filter 900 may be disassembled and assembled from and with adjacent components by clamps 101 to facilitate cleaning.

In addition, the overall size and volume of the cell extruder A of the present invention may be appropriately adjusted by the clamps 101 to be assembled, so that it is easy to be handled in the biological safety cabinet having a limited space.

In addition, the membrane filter installed into the filter holder 500 is provided in the filter holder 500 in the order of decreasing the pore size, and the membrane filters may be provided in the cell extruder at the same time or may be sequentially replaced. For example, the pore size of the membrane filter may be 10 µm, 8 µm, and 5 µm.

The operation of the cell extruder according to the present invention will be described to be divided into preparing, setting, operating, and cleaning with reference to FIGS. 1 and 2.

### Preparing

1) Cell extruder of the present invention provided with 47 mm disk holder
2) Sample: Adipose derived stem cell (5E + 6 cells/ml, total 20 ml)
3) Reagent: PBS (PHOSPHATE BUFFERED SALINE)
4) Consumables required: Membrane filter (whatman PC membrane filter 10, 8, 5 µm), pipette, petri dish

### Setting

1) Prepare the cell extruder A of the present invention of FIGS. 1 and 2 sterilized with an autoclave.
2) Fill a petri dish with a small amount of reagent and soak the membrane filter with a 10 µm pore size.
3) Insert and then lock the membrane filter soaked with the reagent into the filter holder 500.
4) Sequentially mount the cell extruder A of the present invention on the stand 150.
5) Mount a sample container on an end of the filter holder 500.
6) Turn on the pressure gauge 700.
7) Connect a tube supplying gas to the regulator 200. At this time, the input valve 300 is in a closed state.
8) Supply the gas to the regulator 200 to maintain the gas at a set pressure of 50 psi.

### Operating

1) Open the injection line 130 of the pressure vessel 100 and dispense 20 ml of the reagent using a pipette.
2) Close the injection line 130 and close the exhaust valve 400.
3) Open the input valve 300 to supply the gas to the pressure vessel 100.
4) Validate whether the reagent comes out at a constant rate (validation method: requiring 3 seconds based on 20 ml of PBS).
5) Remove the internal pressure of the cell extruder A of the present invention including the pressure vessel 100 by closing the input valve 300 and opening the exhaust valve 400 when the conditions of step 4) above are satisfied.
6) Open the injection line 130 of the pressure vessel 100.
7) Resuspend a sample prepared in a total of 20 ml at a concentration of 5E + 6/ml using a pipette and then dispense the sample into the pressure vessel 100 through the injection line 130.
8) Close the injection line 130 and close the exhaust valve 400.
9) Open the input valve 300 to supply the gas to the pressure vessel 100.
10) Validate whether the sample is extruded and comes out.
11) Remove the internal pressure of the cell extruder A of the present invention including the pressure vessel 100 by closing the input valve 130 and opening the exhaust valve 400.
12) Open the injection line 130 of the pressure vessel 100 again.
13) Dispense the extruded sample into the pressure vessel 100 through the injection line 130 using a pipette again.
14) Repeat steps 6) to 13) twice to perform the extruding three times per membrane filter of one size.
15) Separate the filter holder 500.
16) Remove a membrane filter of 10 µm previously installed in the filter holder 500.
17) Soak a membrane filter having a next size of 8 µm in the reagent in the petri dish.
18) Insert and then lock the membrane filter of 8 µm into the filter holder 500.
19) Reconnect the filter holder 500 to the cell extruder of the present invention.
20) Repeat steps 6) to 14) above.
21) Soak a membrane filter having a next size of 5 µm in the reagent in the petri dish.
22) Repeat steps 6) to 14) above.
23) Filter the sample that has been extruded using a 0.2 µm steri-cup.
24) Store a filtered CDV sample collected in a 50 ml conical tube at 4°C (short-term storage) or - 80°C (long-term storage) for the next step (purification or gUC).

### Cleaning

1) Separate the cell extruder A of the present invention from the stand 150 and then disassemble all clamps 101, and clean the entire configuration by immersing in 0.2 M NaOH for 30 minutes, followed by rinsing with water. At this time, the exhaust filter 900 is not cleaned.
2) Reconnect and assemble the cleaned cell extruder A of the present invention through the clamps 101.
3) Wrap the entire cell extruder A of the present invention with aluminum foil.
4) Sterilize the cell extruder A through an autoclave.
5) Put the sterilized extruder in a bench to perform UV sterilization.

As described above, although the preferred exemplary embodiments of the present invention have been described in detail, the technical scope of the present invention is not limited to the above-described exemplary embodiments and should be interpreted according to the appended claims. At this time, those skilled in the art will understand that many modifications and variations can be made without departing from the scope of the present invention.

## Claims

1. A cell extruder comprising:
a pressure vessel in which a sample is dispensed;
a regulator for adjusting a set pressure of gas injected into the pressure vessel and then maintaining a constant pressure;
an input valve for opening and closing the injection of gas into the pressure vessel;
an exhaust valve for removing an internal pressure of the pressure vessel;
a filter holder which is provided with a membrane filter, so that a sample in the pressure vessel is fed through by the gas and crushed into small pieces; and
a collection container in which the extruded sample is stored.

2. The cell extruder of claim 1, further comprising:
a pressure gauge for measuring the pressure of the pressure vessel.

3. The cell extruder of claim 2, further comprising:
a diaphragm valve that opens a flow path to the filter holder only when the gas pressure acts on a diaphragm to reach the set pressure.

4. The cell extruder of claim 3, wherein the pressure vessel, the regulator, the input valve, the exhaust valve, the filter holder, the collection container, the pressure gauge, and the diaphragm valve are disassembled and assembled from and with adjacent components by clamps.

5. The cell extruder of claim 1, wherein the membrane filters are provided in an order of decreasing a pore size.

6. The cell extruder of claim 5, wherein the membrane filters are provided at the same time or sequentially replaced in the order of decreasing the size.

7. The cell extruder of claim 1, wherein the pressure vessel and the filter holder are connected to each other by a pipe provided with a nozzle.

8. The cell extruder of claim 7, wherein a diameter ratio of the nozzle and the filter holder is 1 : 3 to 1 : 30.

9. The cell extruder of claim 1, wherein the gas is at least one selected from the group consisting of nitrogen gas, compressed air, carbon dioxide, and inert gas.

10. A manufacturing method of cell-derived extracellular vesicles comprising applying the cell extruder of any one of claims 1 to 9 to cells.

11. A manufacturing method of extracellular vesicles comprising:
1) supplying a sample containing cells to the cell extruder of any one of claims 1 to 9;
2) extruding the sample by driving the cell extruder of step 1); and
3) obtaining the sample extruded in step 2).

12. The manufacturing method of extracellular vesicles of claim 11, wherein step 2) includes
i) dispensing the sample to a pressure vessel;
ii) supplying gas to the pressure vessel by opening an input valve; and
iii) passing the sample through a membrane filter in a filter holder from the pressure vessel through a nozzle in a pipe.

13. The manufacturing method of extracellular vesicles of claim 12, wherein the gas is supplied at a pressure of 20 to 100 psi.

14. The manufacturing method of extracellular vesicles of claim 12, wherein step iii) includes passing the sample through the membrane filters sequentially in an order of decreasing a pore size.

15. The manufacturing method of extracellular vesicles of claim 14, wherein the membrane filters are provided at the same time or replaced sequentially in the order of decreasing the size.

16. Cell-derived extracellular vesicles manufactured by the method of claim 10.
